# EUROPEAN PATENT APPLICATION

(11) **EP 2 080 763 A1**
(43) Date of publication of application: **22.07.2009**
(21) Application number: 08000899.8
(22) Date of filing: 18.01.2008
(51) Int. Cl.: C07D 493/04, C07D 493/08, A61K 31/337, A61P 35/00

(54) **Crystalline form I of ortataxel**

(71) Applicant: INDENA S.p.A., 20139 Milano (IT)
(72) Inventor: Ciceri, Daniele, 20139 Milano (IT); Sardone, Nicola, 20139 Milano (IT); Gabetta, Bruno, 20139 Milano (IT); Ricotti, Maurizio, 20139 Milano (IT)
(74) Representative: Minoja, Fabrizio

(57) **Abstract**

The present invention relates to an anhydrous, non-solvated crystalline form of Ortataxel (I) 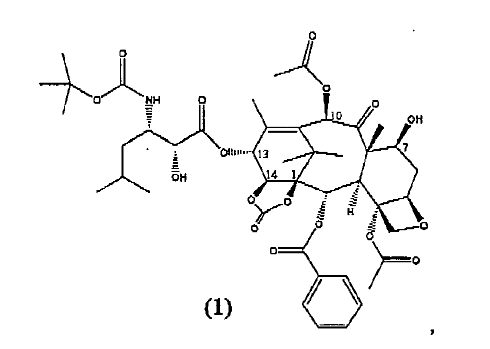 herein referred to as Form I. This form is characterized by very low tendency to absorb humidity, ease of isolation and prolonged chemical and physical stability. Therefore, it can be advantageously used for the preparation of pharmaceutical compositions for the treatment of cancer.

## Description

### Field of the invention

The present invention relates to a novel crystalline form of Ortataxel [13-(N-Boc-β-isobutylserinyl)-14-β-hydroxybaccatin III 1,14-carbonate] and to methods for the preparation thereof.

### Background of the invention

Ortataxel (1) is an antitumor compound particularly active against breast, lung, ovary, colon, prostate, kidney and pancreas tumors, even in case of resistance to known antitumor agents such as adriamycin, vinblastine and some platinum derivatives.

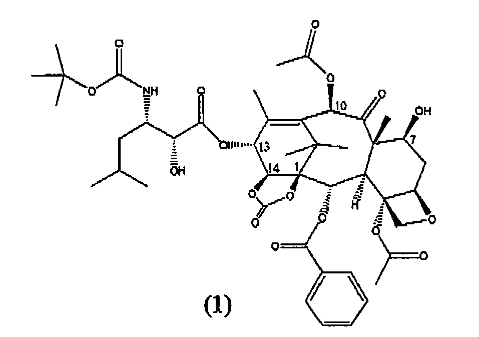

Ortataxel can be prepared according to the methods described in U.S. 7,232,916, in U.S. 6,737,534 and in U.S. 6,906,101. These patents disclose in the examples a final purification step consisting of crystallization from a mixture of acetone and hexane, which gives Ortataxel in the form of a solvate with an acetone content ranging from 4.5 to 6.5%.

The XRPD of the acetone solvate shows distinctive peaks at approximately 7.9, 9.8, 10.6, 10.9, 14.6, 16.9, 19.7, 21.3 deg 2-theta. The DSC curve shows an endothermic peak with onset at about 164°C due to melting and release of the crystallization solvent (confirmed by a weight loss of about 5.0% in TG/DTA) and a weak exothermic peak with maximum at about 212°C followed by an intense endothermic peak with maximum at about 247°C due to melting and incipient decomposition. The IR shows characteristic absorption frequencies at 3521, 3321, 2971, 2953, 1826, 1762, 1706, 1526, 1366, 1238, 1165, 1072, 723 cm⁻¹.

It is well known that volatile impurities in active pharmaceutical ingredients must comply with ICH (International Conference Harmonization) guidelines (Q3C); in this specific case an acetone content from 4.5 to 6.5% would not be allowed. Thus, it would be desirable to find a stable crystalline form of Ortataxel which does not contain residual solvents in amounts unacceptable from a regulatory point of view. Such crystalline form should also be chemically and thermodynamically stable, i.e. it should keep the same quality during storage, and should be obtainable through a reproducible method.

### Disclosure of the invention

It has now been found that Ortataxel can exist in an anhydrous, non-solvated crystalline form, herein after referred to as Form I, which is the subject of the present invention. Form I melts with degradation at 248°C, while the acetone solvate melts with solvent release at 165°C.

Besides its thermodynamic stability, Form I shows other advantages with respect to the pseudopolymorphic acetone solvate: absence of crystallization solvents within its crystal lattice; very low tendency to adsorb humidity; ease of isolation by filtration or centrifugation and chemical and physical stability for at least 36 months.

According to a preferred embodiment of the invention, the preparation of Form I is carried out dissolving Ortataxel, for example Ortataxel acetone solvate obtained according to the synthetic procedures described in the above-cited patents, in suitable amounts (usually 5-12 mL/gₒᵣₜₐₜₐₓₑₗ) of a protic organic solvents such as methanol, ethanol or isopropanol, preferably ethanol, containing 0.01-0.03% (w/v) of an organic acid such as acetic or ascorbic acid, preferably citric acid, in order to avoid partial epimerisation at carbon 7, followed by addition of a suitable amount of water (usually 8-20 mL/gₒᵣₜₐₜₐₓₑₗ); preferably, the ethanol/water ratio is 0.6. The amount of organic acid in the resulting mixture ranges between 0.005-0.03 (w/v). Stirring the mixture at a variable temperature, preferably 35 - 45°C for at least 24 hours, leads to Form (I).

Form (I) can also be prepared stirring Ortataxel, for example Ortataxel acetone solvate, in straight or branched C₅-C₈ alkanes, preferably heptanes, for 5 days, between 0°C and the boiling point of the alkane used, but preferably at 20-30°C.

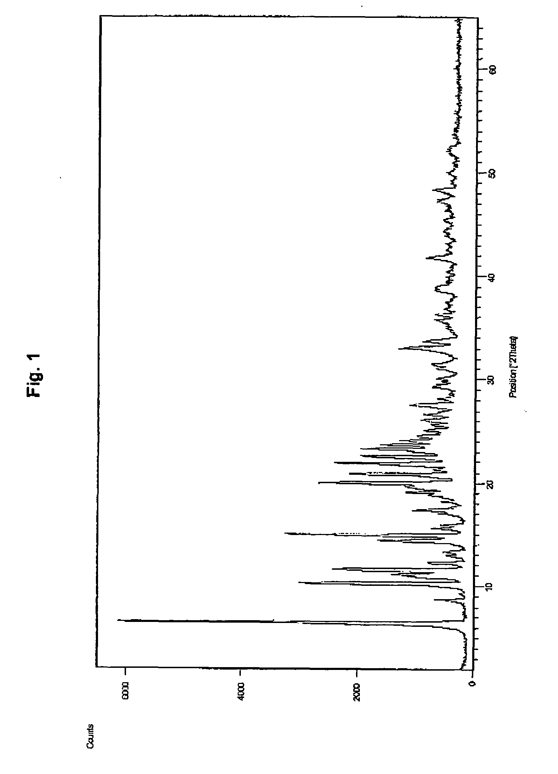

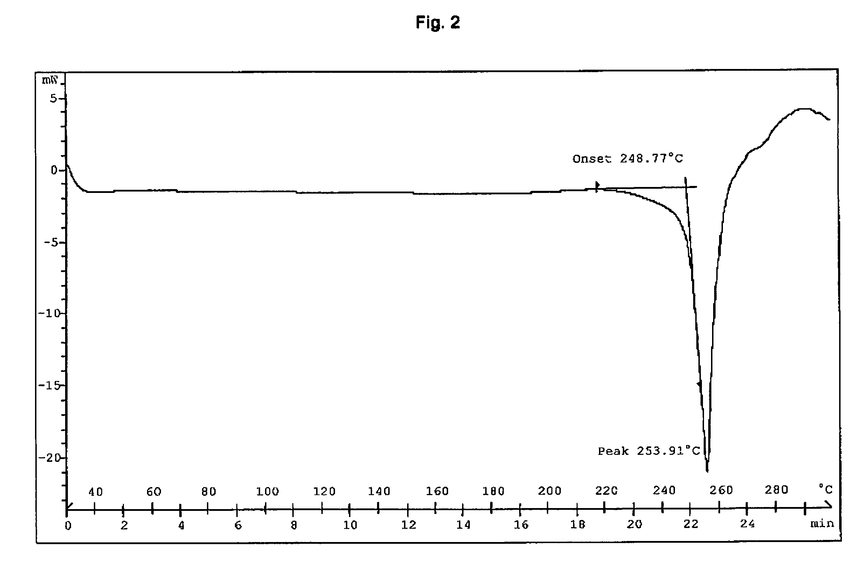

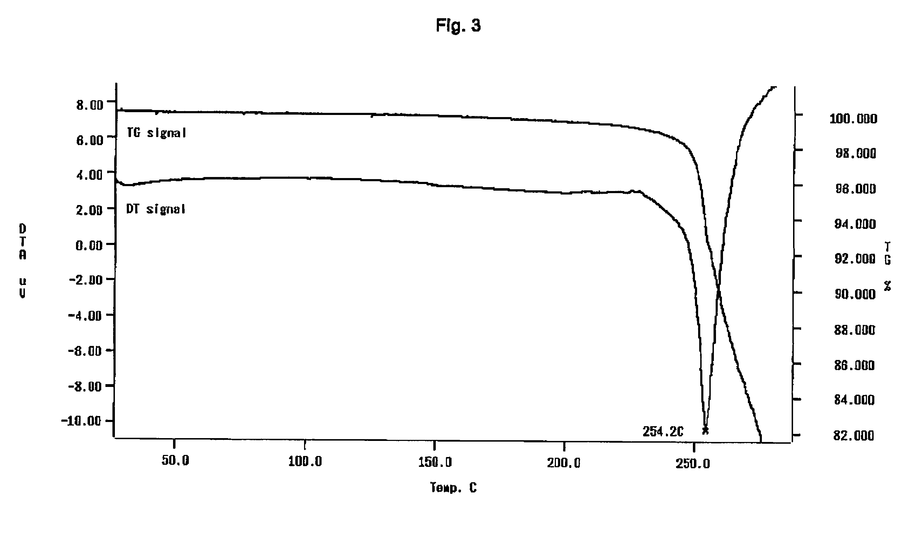

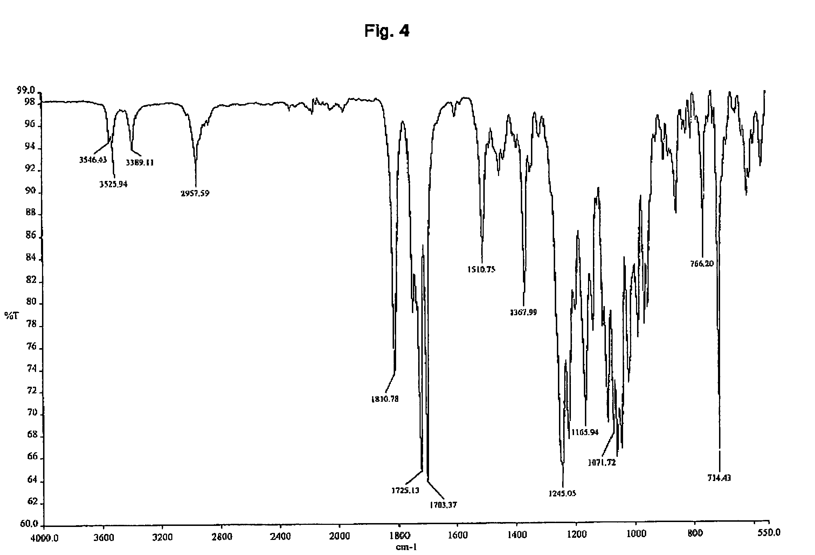

X-ray powder diffraction (XRPD), differential scanning calorimetry (DSC), thermogravimetry and differential thermal analysis (TG/DTA), Fourier-transform infrared spectroscopy (FTIR), dynamic vapour sorption (DVS) and optical microscopy (figures 1-5b) allow to differentiate Form I from the acetone solvate obtainable according to the prior art methods.

Ortataxel crystalline Form I can be advantageously used for the preparation of pharmaceutical compositions for the treatment of cancer. Therefore, a further object of the present invention are pharmaceutical compositions containing Ortataxel crystalline Form I in admixture with pharmaceutically acceptable carriers and/or ingredients, for example those disclosed in "Remington's Pharmaceutical Sciences", Mack Publishing Co., N.Y., USA.

The invention is now illustrated in greater detail in the following experimental section.

### Description of the figures

Figure 1: x-ray powder diffraction pattern of Form I shows a crystalline structure with useful distinctive peaks at approximately 6.5, 8.7, 10.3, 11.6, 14.4, 15.0, 20.0, 21.9, 22.5, 23.3, 23.6, 27.5, 33.0, 38.5, 41.8, 48.1, deg 2-theta.
Figure 2: the DSC curve of Form I exhibits as a solely feature a melting endotherm with maximum at approximately 254°C.
Figure 3: the TG/DT analysis of Form I confirms the DSC analysis showing a DT profile characterized by a steady baseline between 30 and 250°C and a melting peak with maximum at about 254°C. A very low weight loss from 30 to 120°C (about 0.1 %) in the TG profile, coherent with a highly crystalline product, free of moisture and residual organic solvents, is followed by a massive weight loss which takes place upon melting due to a degradative reaction.
Figure 4: the IR spectrum of Form I shows absorption frequencies at 3546, 3525, 3389, 2957, 1810, 1750, 1725, 1703, 1510, 1367, 1245, 1223, 1165, 1139, 1071, 965, 954, 896, 856, 766, 714 cm⁻¹.

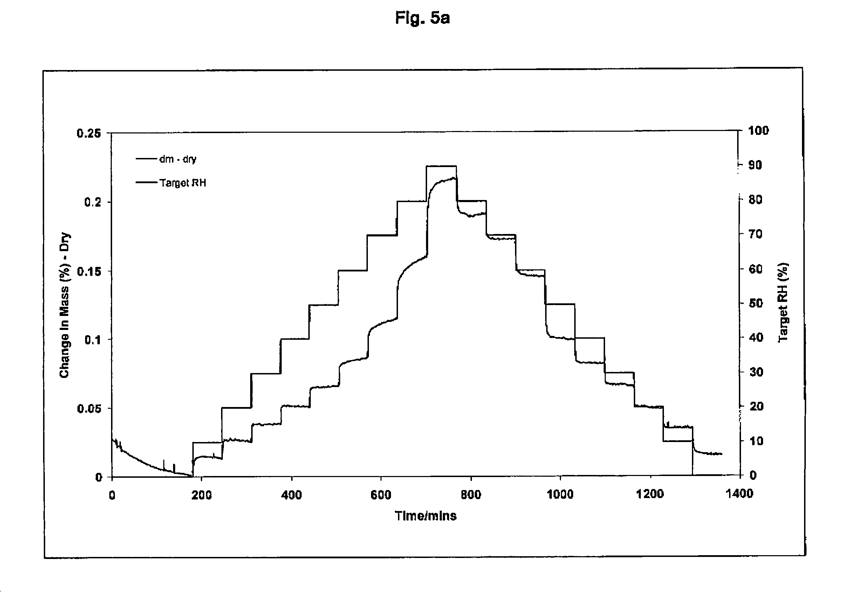

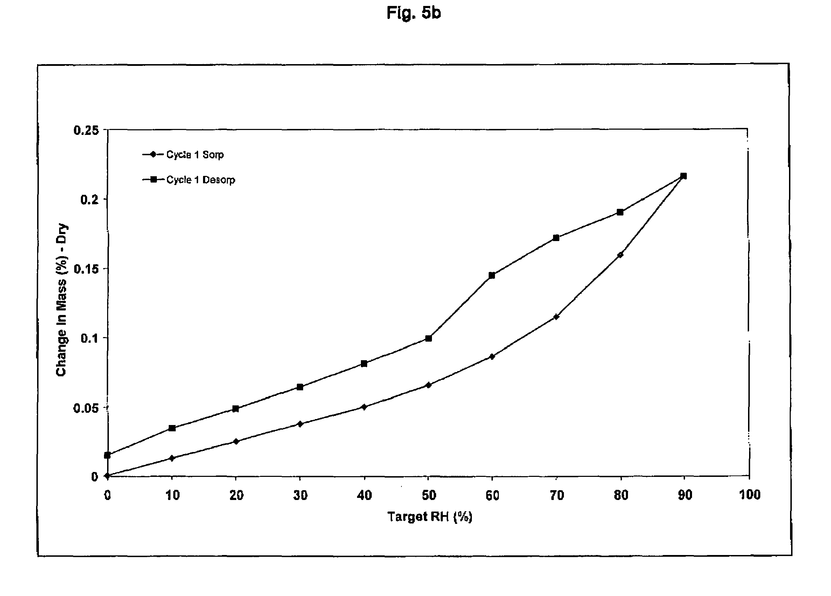

Figures 5a and 5b: the DVS analysis shows that the equilibrium moisture uptake is relatively low (about 0.2% of the dry mass at 90% RH), which is consistent with a highly crystalline form. In most cycles, the equilibrium is reached quickly (about 1300 minutes for a complete sorption/desorption cycle) and the sorption and desorption kinetics are very regular. The corresponding isotherm plot is shown in fig 5b: the sorption/desorption cycle shows an almost closed hysteresis loop, indicating a reversible process. The sorption part of the isotherm is below the desorption part, which is characteristic of adsorption of water into the bulk of the powder. During the kinetically controlled adsorption process there is no evidence of dissolution phenomena or changes in the solid form. Neither re-crystallisation nor irreversible morphological changes take place when the sample is exposed to humidity.

Optical microscopy shows that solid Form I is constituted by transparent prismatic columnar crystals.

Hence, these data clearly indicate that Ortataxel (1) in its polymorphic Form I is easily distinguishable from the pseudopolymorphic acetone solvate by XRPD, DSC, IR and any analysis for the solvent content (thermogravimetry or gas-chromatography).

### Experimental section

### Materials and methods

X-ray powder diffraction patterns were recorded on a Philips PW1800 diffractometer. The x-ray generator was operated at 45 kV and 40 mA, using the Cu Kα line as radiation source. The sample was packed on a suitable slit and the irradiated length was 10 mm. Data were collected between 2 and 65 deg 2-theta with a step size of 0.02 deg 2-theta.

Measurements of differential scanning calorimetry (DSC) were performed using a Mettler TC15 System equipped with a DSC20 measuring cell, using closed aluminum crucibles (40 µl volume) with a pinhole. Heat flow was recorded from 30 to 300°C with a linear heating rate of 10°C/min under a 50 ml/min nitrogen flow. About 5 mg of powder was used for each measurement.

Thermogravimetry and differential thermal analyses (TG/DTA) were performed using a Seiko TG/DTA6200 simultaneous system using open aluminum pans (40 µl volume). The TG/DT signals were recorded from 30 to 300°C with linear heating rate (10°C/min) under a 200 ml/min nitrogen flow. About 10 mg powder was used for each measurement.

Fourier-transform infrared spectroscopy spectra (FTIR) were recorded with the ATR technique using a Fourier-transform spectrometer Perkin Elmer Spectrum One. The spectra were the result of the acquisition and transformation of 16 co-added scans in the 4000-550 cm⁻¹ spectral region at a resolution of 4 cm⁻¹.

The measurements of dynamic vapour sorption were carried out with the Dynamic Vapour Sorption System DVS-1. The analyses were run at a fixed temperature of 25°C, with a sample size of few milligrams. The sample was dried for 6 hours under a continuous flow of dry air (Relative Humidity RH < 0.1%) to establish the dry mass (preconditioning step).

The relative humidity was then increased from 0% to 90% RH (in 10% RH steps) and then decreased in a similar way. The instrument was run in a dm/dt mode (mass variation over time variation) to decide when equilibrium was reached; a fixed dm/dt value of 0.0005%/min was selected. The dm/dt criterion allows the DVS software to automatically terminate each relative humidity step. When the rate of change of mass falls below this threshold, umidity moves on to the next level. A maximum stage time of 6 hours along with a minimum of 1 hour were chosen.

Optical microscopy analyses were performed using a transmitted-light microscope Zeiss Axioskop. For each analysis a little amount of sample was dispersed in silicone oil, mounted on a specimen slide and covered with a micro cover glass. The observations were carried out under appropriate conditions of illumination, contrast and magnification.

### Example 1

Ortataxel acetone solvate (110 g), prepared as described in U.S. No 7,232,916, was dissolved in 95% ethanol (880 mL) containing citric acid (143 mg) at 50°C. Cold purified water (1430 mL) was added over 15 minutes. The initially slurry was stirred at 40°C for 24 hours providing eventually an easily stirrable suspension. The mixture was cooled to 20°C and the white solid was filtered off. The solid was washed with 330 mL of 35% ethanol and dried under vacuum at 50°C for 24 hours affording 103.3 g of a white solid having the characteristic XRPD, DSC, IR reported in figures 1-3 respectively.

### Example 2

Ortataxel acetone solvate (5.0 g) was suspended in heptane (200 mL) and stirred for 5 days at 26°C. The solid was filtered off and dried under vacuum at 50°C for 24 hours. A white product having the characteristic XRPD, DSC, IR reported in figures 1-3 respectively was obtained.

## Claims

1. Crystalline Form I of Ortataxel [13-(N-Boc-β-isobutylserinyl)-14-β-hydroxybaccatin III 1,14-carbonate] (1) 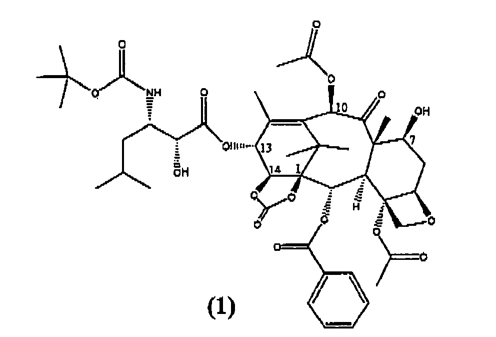 having an XRPD spectrum **characterized by** the following peaks: 6.5, 8.7, 10.3, 11.6, 14.4, 15.0, 20.0, 21.9, 22.5, 23.3, 23.6, 27.5, 33.0, 38-5, 41.8, 48.1, deg 2-theta.

2. A process for preparing the crystalline form of Ortataxel of claim 1, which comprises stirring for at least 24 hours Ortataxel in a mixture of a protic organic solvent and water at a temperature ranging between 0°C and 60°C in the presence of 0.005-0.03% w/v organic acid.

3. A process according to claim 2 in which the protic organic solvent is selected from methanol, ethanol and isopropanol.

4. A process according to claim 3 wherein the protic organic solvent is ethanol.

5. A process according to claim 2 in which the ethanol-water ratio is 0.5 to 0.7.

6. A process according to any one of claims 2 - 5 in which the temperature ranges between 35 and 45°C.

7. A process according to any one of claim 2 - 6 in which the organic acid is citric acid or ascorbic acid.

8. A process according to claim 7 in which the organic acid is citric acid.

9. A process for preparing the crystalline Form I of Ortataxel of claim 1, which comprises stirring Ortataxel in a straight or branched C₅-C₉ alkane or in a mixture thereof at a temperature between 0°C and the boiling point of the alkane.

10. A process according to claim 9 in which the alkane is heptane.

11. A process according to claim 8 or 9 in which the temperature ranges between 20 and 30°C.

12. Pharmaceutical compositions containing the Ortataxel crystalline Form I of claim 1 in admixture with suitable excipients and/or carriers.

13. Use of the Ortataxel crystalline Form I of claim 1 for the preparation of pharmaceutical compositions for the treatment of cancer.
